# EUROPEAN PATENT SPECIFICATION

(11) **EP 2 877 146 B1**
(45) Date of publication and mention of the grant of the patent: **19.09.2018**
(21) Application number: 13740295.4
(22) Date of filing: 24.07.2013
(51) Int. Cl.: A61K 8/34, A61K 8/41, A61Q 5/12, A61K 8/04

(54) **PROCESS**
VERFAHREN
PROCÉDÉ

(30) Priority: 27.07.2012 EP 12178171
(43) Date of publication of application: 03.06.2015
(73) Proprietor: Unilever PLC, London, Greater London EC4Y 0DY (GB); Unilever N.V., 3013 AL Rotterdam (NL)
(72) Inventor: CASUGBO, Christia, Bebington Wirral Merseyside CH63 3JW (GB); FLANAGAN, Mark, Bebington Wirral Merseyside CH63 3JW (GB); HOUGH, John, Alan, Bebington Wirral Merseyside CH63 3JW (GB); NAUGHTON, John, Michael, Bebington Wirral Merseyside CH63 3JW (GB); SERRIDGE, David, Bromborough Wirral Merseyside CH62 2FD (GB)
(74) Representative: Chisem, Janet
(86) International application number: PCT/EP2013/065648
(87) International publication number: WO 2014/016354

(56) References cited:
- EP-A1- 2 460 508
- WO-A1-2008/055816
- WO-A2-2007/136708

## Description

The present invention relates to processes for making improved conditioning compositions.

EP-A1-2 460 508 (P&G) discloses a hair conditioning composition comprising a cationic surfactant, a high melting point fatty compound and an aqueous carrier. The method for manufacture involves preparing a premix comprising the cationic surfactants and fatty compounds, wherein the temperature of the premix is higher than a melting point of the fatty compound.

WO 2008/055816 (Unilever) discloses conditioning shampoo compositions comprising a gel network that comprises a quaternary ammonium compound and a C12-C22 fatty alcohol. In the examples, cetyl alcohol is added to water at 65C with high speed stirring and then followed by adding CTAC to make a uniform dispersion at 65C. This is then added to aqueous solution at room temperature with moderate stirring.

WO 2007/136708 (P&G) discloses hair care compositions comprising an aminosilicone. The method of preparation comprises heating de-ionised water to 85C and mixing in cationic surfactants and fatty compounds. Water is maintained at 85C until the components are homogenised then the mixture is cooled to around 55C and maintained at this temperature to form a gel matrix.

Despite the prior art there remains a need for improved conditioning compositions.

According, and in a first aspect, there is provided a process for making a conditioning gel phase according to claim 1.

The comelting of the fatty alcohol and the cationic surfactant forms an isotropic phase. This means that the development of structure, i.e. the formation of the lamellar conditioning gel phase, can be controlled by the temperature and rate of mixing of the comelt and the water. The conditioning composition ultimately made using such conditioning gel phase has superior conditioning capability.

The conditioning compositions made using a conditioning gel phase of the invention are superior products to those made mixing the water, fatty alcohol and cationic surfactant at around 70C. Specifically, the superiority manifests itself in superior next day conditioning benefits where one would expect superior conditioning benefits to be due to increased deposition of solids thus leaving the hair lank and greasy the following day.

The improvement thus resides in the balance of thermal energy at the point of mixing the water with the comelt.

If the water is too cold then the comelt solidifies resulting in a poorly mixed system and this ultimately provides a composition of low viscosity. If the temperature of the water is too high then it is also too high at the point of mixing with the comelt and so forms vesicles. This also gives rise to lower viscosity in the conditioning composition formed with the resulting conditioning gel phase.

Preferably, the water in the second vessel is maintained at 56-60°C and more preferably at 57-59°C.

Preferably, the comelt comprises from 45-90% wt. comelt fatty alcohol.

Preferably, the fatty alcohol comprises from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is particularly preferable.

The level of fatty alcohol in the conditioner of the invention (not just the conditioning gel phase) will generally range from 0.01 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

Preferably, the comelt comprises from 10-40% wt. of the comelt cationic component.

Suitable conditioning surfactants include those selected from cationic surfactants, used singly or in admixture. Preferably, the cationic surfactants have the formula N⁺R¹R²R³R⁴ wherein R¹, R², R³ and R⁴ are independently (C₁ to C₃₀) alkyl or benzyl. Preferably, one, two or three of R¹, R², R³ and R⁴ are independently (C₄ to C₃₀) alkyl and the other R¹, R², R³ and R⁴ group or groups are (C₁-C₆) alkyl or benzyl. More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or - COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable.

A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Preferably, the cationic component of the comelt comprises from 0-70% wt. cationic component, cationic surfactants have the formula N⁺R¹R²R³R⁴ as described above, more preferably from 30-60% wt. cationic component.

Another example of a class of suitable cationic surfactants for use in the invention, either alone or together with one or more other cationic surfactants, is a combination of (i) and (ii) below:
(i) an amidoamine corresponding to the general formula (I):

   R1CONH(CH2)mN(R2)R3

   in which R¹ is a hydrocarbyl chain having 10 or more carbon atoms, R² and
   R³ are independently selected from hydrocarbyl chains of from 1 to 10 carbon atoms, and m is an integer from 1 to about 10; and
(ii) an acid.

As used herein, the term hydrocarbyl chain means an alkyl or alkenyl chain. Preferred amidoamine compounds are those corresponding to formula (I) in which R¹ is a hydrocarbyl residue having from about 11 to about 24 carbon atoms,
R² and R³ are each independently hydrocarbyl residues, preferably alkyl groups, having from 1 to about 4 carbon atoms, and m is an integer from 1 to about 4.

Preferably, R² and R³ are methyl or ethyl groups.

Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyl-diethylamine, palmitamidoethyldiethylamine, palmitamidoethyldimethylamine, behenamidopropyldimethyl-amine, behenamidopropyldiethylmine, behenamidoethyldiethyl-amine, behenamidoethyldimethylamine, arachidamidopropyl-dimethylamine, arachidamidopropyldiethylamine, arachid-amidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

Acid may be any organic or mineral acid which is capable of protonating the amidoamine in the conditioner composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, lactic acid and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate more than 95 mole% (293 K) of the amidoamine present.

Should an amidoamine of the type described herein be present then the corresponding acid component will not be present in the comelt. Instead it will be present in the second vessel with the water. Preferably, the water in the second vessel comprises protonating component at from 0.01 to 3% wt.

Accordingly, where the invention requires from 10-40% wt. comelt cationic surfactant, the cationic surfactant component may comprise amidoamine which is not protonated, i.e. it will not be cationic charged but will become protonated when added to the water and hence the protonating material contained therein.

Preferably, the cationic surfactant component of the comelt comprises from 0-70% cationic component, amidoamine corresponding to formula (I), more preferably from 30-60% wt. cationic surfactant component.

In conditioning compositions of the invention (not merely the conditioning gel phase), the level of cationic surfactant will generally range from 0.01 % to 10%, more preferably 0.05 % to 7.5%, most preferably 0.1 % to 5% by weight of the composition.

Preferably, the comelt is maintained at a melting point sufficient to maintain the fatty alcohol in a liquid phase. Preferably, the comelt is maintained at from 80-85°C.

Preferably, the temperature of the mixture of the comelt and the water is controlled such that it is maintained from 56-65°C, prefer from 58-62°C, more preferably 60°C during mixing.

Preferably, the contents of the first vessel are gradually added to the second vessel and passed through a mixer with rotor tip speed of 10-34, preferably from 21-27 and especially preferably 24 ms-1.

Preferably, this mixing step is maintained for sufficient time for 95% v/v of the comelt/water mixture to have passed through the mixer at least once. Typically, this is from 20-40 minutes.

In a second aspect there is provided a process for manufacturing a conditioning composition by forming a conditioning gel phase obtained by the first aspect and then adding any remaining ingredients. Typical remaining ingredients include fragrances, silicones, fibre actives or other benefit agents.

Preferably, the conditioning composition is passed through a mixer with rotor tip speed of 10-34, preferably from 21-27 and especially preferably 24 ms-1 one more time after the remaining ingredients have been added.

Conditioning compositions of the invention or using conditioning gel phases of the invention also deposit silicone better than conventionally made conditioning compositions.

Accordingly, the compositions of the invention can contain, emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst at 25 °C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the shampoo compositions of the invention will typically have an average silicone droplet size in the composition of less than 30, preferably less than 20, more preferably less than 10 micron, ideally from 0.01 to 1 micron. Silicone emulsions having an average silicone droplet size of 0.15 micron are generally termed microemulsions.

Emulsified silicones for use in the conditioner compositions of the invention will typically have an size in the composition of less than 30, preferably less than 20, more preferably less than 15. Preferably the average silicone droplet is greater than 0.5 micron, more preferably greater than 1 micron, ideally from 2 to 8 micron.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

The total amount of silicone is preferably from 0.01 wt% to 10 %wt of the total composition more preferably from 0.1 wt% to 5 wt%, most preferably 0.5 wt% to 3 wt% is a suitable level.

### EXAMPLES

| **INCI name** | **Active Level** | **A** | **1** |
|---|---|---|---|
| Behentrimonium Chloride | 70 | 2.86 | 2.28 |
| Dimethicone/amodimethicone/ Cetrimonium Chloride | 70 | 4.29 | 4.29 |
| Cetearyl Alcohol | 100 | 4 | 3.2 |
| DMDM Hydantoin | 55 | 0.1 | 0.1 |
| Potassium Chloride | 100 | 0.1 | 0.1 |
| Parfum | 100 | 0.6 | 0.6 |
| Methylisothiazolinone | 100 | 0.04 | 0.04 |
| Methylchloroisothaizolinone | | | |
| Aqua | 100 | To 100 | To 100 |

Formulation A is made by standard processes involving heating the fatty alcohol and cationic surfactant in water at around 70°C while formulation 1 is made by adding cationic surfactants to fatty alcohol and stir at 85°C.

Gradually add this mixture to water, containing other ingredients, typically at 55°C, but at a temperature tailored to the composition to ensure mixture temperature is 60°C, this temperature maintained by external heating if required, and stir.

Cool this towards ambient by adding more water, and other ambient temperature ingredients, and use of external cooling if required, and stir.

The compositions have different levels of conditioning active to demonstrate the improved conditioning performance of the composition made by the claimed process.

| | **A** | **1** |
|---|---|---|
| No of Panellists | 32 | 32 |

| **Conditioner in-use** | | |
|---|---|---|
| Con thickness | 49.84 C | 64.88 B |
| ease application | 70.17 C | 80.12 A |
| ease of spread | 72.73 B | 79.16 A |
| detangling | 67.89 B | 73.74 A |

| **Dry** | | |
|---|---|---|
| dry residue | 7.20 b | 9.48 ab |

| **Next day** | | |
|---|---|---|
| ND greasiness | 17.43 BC | 13.31 C |
| ND static | 9.68 A | 7.27 BCD |
| ND clean feel | 69.41 AB | 71.33 A |
| ND conditioning | 65.65 B | 70.47 A |

Panel data with approx 40 panellists with damaged hair. Assessment via line scale.

Panel data with approx 40 panellists with dry damaged hair. Assessment via line scale. (5 different products tested in total vs. control).

The data shows that using a better process we have a thicker product despite having lower total solids (i.e. FA and BTAC). The ingredients are being used more efficiently.

In addition, the product is both significantly more conditioning than the control as well as feeling significantly more clean next day - unusual because there is usually a trade off (more conditioning= heavier) again, despite having a lower level of solids, i.e. conditioning active. One would have expected that a composition which provided improved conditioning benefits immediately post application would achieve this through increased deposition. However, if this were the case, the next day benefits would be markedly reduced.

## Claims

1. Process for making a conditioning gel phase comprising:
- forming a 'comelt' in a first vessel comprising fatty alcohol and cationic component and 0-15% wt. comelt of water (A)
- adding the 'comelt' to a second vessel containing water at 50-60°C (B)
- mixing
wherein the temperature of the mixture of the comelt and the water in the second vessel (B) is controlled such that it is maintained from 56-65°C, preferably from 58-62°C, more preferably 60°C, wherein the fatty alcohol has from 8 to 22 carbons and wherein the cationic component comprises from 0-70% wt. cationic surfactants have the formula N⁺R¹R²R³R⁴, more preferably from 30-60% wt. cationic surfactant component, and wherein R¹, R², R³ and R⁴ are independently (C₁ to C₃₀) alkyl or benzyl.

2. Process according to claim 1 wherein the comelt comprises from 45-90% wt. comelt fatty alcohol.

3. Process according to claim 1 or 2 wherein the comelt comprises from 10-40% cationic surfactant.

4. Process according to any preceding claim wherein the comelt is maintained at a melting point sufficient to maintain the fatty alcohol in a liquid phase (pref 80-85°C).

5. Process according to any preceding claim wherein the contents of the first vessel are gradually added to the second vessel and passed through a mixer with rotor tip speed of 10-34 ms-1.

6. Process according to any preceding claim wherein the second vessel comprises neutralising agent, preferably lactic acid.

7. Process for manufacturing a conditioning composition by forming a conditioning gel phase obtained by any of claims 1-6 and then adding any remaining ingredients.

8. Process according to claim 7 comprising passing the composition through a mixer with rotor tip speed of 10-30 ms-1 .

## Patentansprüche

1. Verfahren zur Herstellung einer konditionierenden Gelphase, umfassend:
- Ausbilden einer "Co-Schmelze" in einem ersten Behälter, umfassend Fettalkohol und kationische Komponente und 0-15 Gew.-% Co-Schmelze von Wasser (A),
- Zugeben der "Co-Schmelze" zu einem zweiten Behälter, der Wasser bei 50-60°C (B) enthält,
- Mischen,
wobei die Temperatur der Mischung der Co-Schmelze und des Wassers in dem zweiten Behälter (B) derartig gesteuert wird, dass sie bei 56-65°C, vorzugsweise bei 58-62°C, bevorzugter bei 60°C, aufrechterhalten wird, wobei der Fettalkohol von 8 bis 22 Kohlenstoffatome umfasst und wobei die kationische Komponente von 0-70 Gew.-% kationische Tenside, die die Formel N⁺R¹R²R³R⁴ aufweisen, bevorzugter von 30-60 Gew.-% kationische Tensidkomponente umfasst und wobei R¹, R², R³ und R⁴ unabhängig (C₁ bis C₃₀)-Alkyl oder Benzyl darstellen.

2. Verfahren nach Anspruch 1, wobei die Co-Schmelze von 45-90 Gew.-% Co-Schmelze-Fettalkohol umfasst.

3. Verfahren nach Anspruch 1 oder 2, wobei die Co-Schmelze von 10-40% kationisches Tensid umfasst.

4. Verfahren nach irgendeinem vorhergehenden Anspruch, wobei die Co-Schmelze bei einem Schmelzpunkt aufrechterhalten wird, der ausreicht, um den Fettalkohol in flüssiger Phase (vorzugsweise 80-85°C) zu halten.

5. Verfahren nach irgendeinem vorhergehenden Anspruch, wobei der Gehalt des ersten Behälters graduell zu dem zweiten Behälter gegeben und durch einen Mischer mit einer Rotorspitzengeschwindigkeit von 10-34 ms⁻¹ geführt wird.

6. Verfahren nach irgendeinem vorhergehenden Anspruch, wobei der zweite Behälter ein neutralisierendes Mittel, vorzugsweise Milchsäure, umfasst.

7. Verfahren zur Herstellung einer konditionierenden Zusammensetzung durch Ausbilden einer konditionierenden Gelphase, erhalten durch irgendeinen der Ansprüche 1-6, und darauf Zufügen aller verbleibenden Bestandteile.

8. Verfahren nach Anspruch 7, umfassend das Strömen der Zusammensetzung durch einen Mischer mit einer Rotorspitzengeschwindigkeit von 10-30 ms⁻¹.

## Revendications

1. Procédé de fabrication d'une phase de gel de conditionnement comprenant :
- la formation d'une 'cofusion' dans une première cuve comprenant un alcool gras et un constituant cationique et 0-15 % en masse de cofusion d'eau (A)
- l'addition de la 'cofusion' dans une seconde cuve contenant de l'eau à 50-60°C (B)
- le mélange
dans lequel la température du mélange de la cofusion et de l'eau dans la seconde cuve (B) est contrôlée de telle sorte qu'elle est maintenue à 56-65°C, de préférence à 58-62°C, encore mieux, à 60°C, dans lequel l'alcool gras présente de 8 à 22 atomes de carbone et dans lequel le constituant cationique comprend 0-70 % en masse de tensioactifs cationiques qui présentent la formule N⁺R¹R²R³R⁴, encore mieux 30-60 % en masse de constituant de tensioactif cationique, et dans lequel R¹, R², R³ et R⁴ sont indépendamment un groupe alkyle en (C₁ à C₃₀) ou benzyle.

2. Procédé selon la revendication 1, dans lequel la cofusion comprend 45-90 % en masse d'alcool gras de cofusion.

3. Procédé selon la revendication 1 ou 2, dans lequel la cofusion comprend 10-40 % de tensioactif cationique.

4. Procédé selon l'une quelconque des revendications, dans lequel la cofusion est maintenue à un point de fusion suffisant pour maintenir l'alcool gras dans une phase liquide (de préférence 80-85°C).

5. Procédé selon l'une quelconque des revendications précédentes, dans lequel les contenus de la première cuve sont progressivement ajoutés dans la seconde cuve et passent à travers un mélangeur avec une vitesse de lame de rotor de 10-34 ms⁻¹.

6. Procédé selon l'une quelconque des revendications précédentes, dans lequel la seconde cuve comprend un agent de neutralisation, de préférence de l'acide lactique.

7. Procédé de fabrication d'une composition de conditionnement pour former une phase de gel de conditionnement obtenue par l'une quelconque des revendications 1-6 et d'addition subséquente des ingrédients restants.

8. Procédé selon la revendication 7 comprenant le passage de la composition à travers un mélangeur avec une vitesse de lame de rotor de 10-30 ms⁻¹.
